# EUROPEAN PATENT SPECIFICATION

(11) **EP 1 850 832 B1**
(45) Date of publication and mention of the grant of the patent: **31.12.2014**
(21) Application number: 06704828.0
(22) Date of filing: 07.02.2006
(51) Int. Cl.: A61K 39/39

(54) **ADJUVANTING MATERIAL**
ADJUVANS-MATERIAL
MATÉRIAU ADJUVANT

(30) Priority: 07.02.2005 AU 2005900518
(43) Date of publication of application: 07.11.2007
(73) Proprietor: Lipotek PTY Ltd, Acton, ACT 0200 (AU)
(72) Inventor: JACKSON, David, C., North Balwyn, Victoria 3104 (AU); PARISH, Christopher, Richard, Campbell, Australian Capital Territory 2 (AU)
(74) Representative: Wichmann, Hendrik
(86) International application number: PCT/AU2006/000147
(87) International publication number: WO 2006/081631

(56) References cited:
- WO-A1-2005/018610
- CHIKH G G ET AL: "Attaching histidine-tagged peptides and proteins to lipid-based carriers through use of metal-ion-chelating lipids" BIOCHIMICA ET BIOPHYSICA ACTA. BIOMEMBRANES, AMSTERDAM, NL LNKD- DOI:10.1016/S0005-2736(02)00618-1, vol. 1567, 23 December 2002 (2002-12-23), pages 204-212, XP004399280 ISSN: 0005-2736
- REITERMANN A ET AL: "LIPOPEPTIDE DERIVATIVES OF BACTERIAL LIPOPROTEIN CONSTITUTE POTENT IMMUNE ADJUVANTS COMBINED WITH OR COVALENTLY COUPLED TO ANTIGEN OR HAPTEN//LIPOPEPTIDE ALS POTENTE IMMUNOADJUVANTIEN UND IMMUNOGENITAET VON LIPOPEPTID-HAPTEN-KONJUGATEN" BIOLOGICAL CHEMISTRY HOPPE-SEYLER, WALTER DE GRUYTER, BERLIN, DE, vol. 370, no. 4, 1 April 1989 (1989-04-01) , pages 343-352, XP008020632 ISSN: 0177-3593
- JACKSON D C ET AL: "A totally synthetic vaccine of generic structure that targets Toll-like receptor 2 on dendritic cells and promotes antibody or cytotoxic T cell responses" PROCEEDINGS OF THE NATIONAL ACADEMY OF SCIENCES OF THE UNITED STATES (PNAS), NATIONAL ACADEMY OF SCIENCE, US LNKD- DOI:10.1073/PNAS.0406740101, vol. 101, no. 43, 26 October 2004 (2004-10-26), pages 15440-15445, XP002498182 ISSN: 0027-8424 [retrieved on 2004-10-15]
- REITERMANN A. ET AL.: 'Lipopeptide derivatives of Bacterial Lipoprotein Constitute Potent Immune Adjuvants Combined with or Covalently Coupled to Antigen or Hapten' BIOL. CHEM. HOPPE-SEYLER vol. 370, 1989, pages 343 - 352, XP008020632
- JACKSON D.C. ET AL: 'A Totally synthetic vaccine of generic structure that targets Toll-like receptor 2 in dendritic cells and promotes antibody or cytotoxic T cell responses' PNAS vol. 101, no. 43, October 2004, pages 15440 - 15445, XP002498182
- SACHT G. ET AL.: 'Activation of nuclear factor-khiB in macrophages by mycoplasmal lipopeptides' EUR. J. IMMUNOL. vol. 28, 1998, pages 4207 - 421, XP003015633
- CHIKH G. ET AL.: 'Attaching histidine-tagged peptides and proteins to lipid-based carriers through 'use of metal-ion-chelating lipids' BIOCHIMICA ET BIOPHYSICA ACTA vol. 1567, 2002, pages 204 - 212, XP004399280
- VAN BROEKHOVEN C. ET AL.: 'Targeting Dendritic Cells with Antigen-Containing Liposomes: A Highly Effective Procedure for Induction of Antitumour Immunity and for Tumour Immunotherapy' CANCER RES. vol. 64, 2004, pages 4357 - 4365, XP002443804

## Description

### FIELD OF THE INVENTION

The present invention relates to compounds and compositions for use in generating immune responses.

### BACKGROUND OF THE INVENTION

Immunotherapy or vaccination are attractive for the prophylaxis or therapy of a wide range of disorders, such as, for example, certain infectious diseases, or cancers. However, the application and success of such treatments are limited in part by the poor immunogenicity of the target antigen. Many peptides, glycopeptides, proteins, glycoproteins, lipids, lipopeptides, carbohydrates etc., are poorly immunogenic. Several techniques are used to enhance the immune response of a subject to an immunogen.

It is known to utilize an adjuvant formulation that is extrinsic to the peptide/protein immunogen (i.e. it is mixed with the immunogen prior to use), such as, for example, complete Freund's adjuvant (CFA), to enhance the immune response of a subject to a peptide/protein immunogen. However, many of the adjuvants currently available are too toxic for use in humans, or simply ineffective.

Lipopeptides, wherein a lipid moiety that is known to act as an adjuvant is covalently coupled to a peptide immunogen, may be capable of enhancing the immunogenicity of an otherwise weakly immunogenic peptide in the absence of an extrinsic adjuvant [Jung et al., Angew Chem, Int Ed Engl 10, 872, (1985); Martinon et al., J Immunol 149, 3416, (1992); Toyokuni et al., J Am Chem Soc 116, 395, (1994); Deprez, et al., J Med Chem 38, 459, (1995); and Sauzet et al., Vaccine 13, 1339, (1995); Benmohamed et al., Eur. J. Immunol. 27, 1242, (1997); Wiesmuller et al., Vaccine 7, 29, (1989); Nardin et al., Vaccine 16, 590, (1998); Benmohamed, et al. Vaccine 18, 2843, (2000); and Obert, et al., Vaccine 16, 161, (1998)]. Suitable lipopeptides show none of the harmful side effects associated with adjuvant formulations, and both antibody and cellular responses have been observed against lipopeptides.

Several different fatty acids are known for use in lipid moieties. Exemplary fatty acids include, but are not limited to, palmitoyl, myristoyl, stearoyl and decanoyl groups or, more generally, any C2 to C30 saturated, monounsaturated, or polyunsaturated fatty acyl group is thought to be useful.

The lipoamino acid N-palmitoyl-S-[2,3-bis(palmitoyloxy)propyl]cysteine, also known as Pam3Cys or Pam3Cys-OH (Wiesmuller et al., Z. Physiol.Chem. 364 (1983), p593), is a synthetic version of the N-terminal moiety of Braun's lipoprotein that spans the inner and outer membranes of Gram negative bacteria. Pam3Cys has the structure of Formula (I):

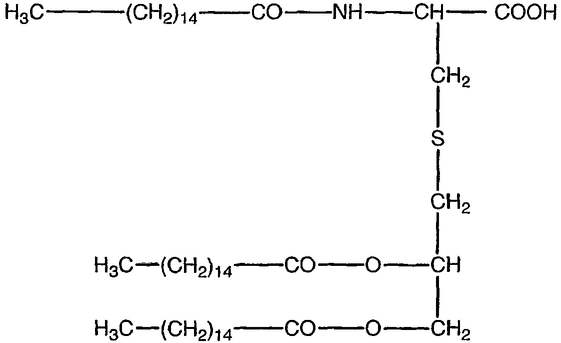

United States Patent No. 5, 700, 910 to Metzger et al (December 23, 1997) describes several N-acyl-S-(2-hydroxyalkyl)cysteines for use as intermediates in the preparation of lipopeptides that are used as synthetic adjuvants, B lymphocyte stimulants, macrophage stimulants, or synthetic vaccines. Metzger et al. also teach the use of such compounds as intermediates in the synthesis of Pam3Cys-OH (Wiesmuller et al., Z. Physiol.Chem. 364, p593, 1983), and of lipopeptides that comprise this lipoamino acid or an analog thereof at the N-terminus.

Pam3Cys has been shown to be capable of stimulating virus-specific cytotoxic T lymphocyte (CTL) responses against influenza virus-infected cells (Deres et al., Nature 342, 561, 1989) and to elicit protective antibodies against foot-and-mouth disease (Wiesmuller et al., Vaccine 7, 29, 1989; United States Patent No. 6,024,964 to Jung et al., February 15, 2000) when coupled to the appropriate epitopes.

Recently, Pam2Cys (also known as dipalmitoyl-S-glyceryl-cysteine or S-[2,3-bis(palmitoyloxy)propyl]cysteine), an analogue of Pam3Cys, has been synthesised (Metzger, J. W., A. G. Beck-Sickinger, M. Loleit, M. Eckert, W. G. Bessler, and G. Jung. 1995. J Pept Sci 1:184.) and been shown to correspond to the lipid moiety of MALP-2, a macrophage-activating lipopeptide isolated from mycoplasma (Sacht, G., A. Marten, U. Deiters, R. Sussmuth, G. Jung, E. Wingender, and P. F. Muhlradt. 1998. Eur J Immunol 28:4207: Muhlradt, P. F., M. Kiess, H. Meyer, R. Sussmuth, and G. Jung. 1998. Infect Immun 66:4804: Muhlradt, P. F., M. Kiess, H. Meyer, R. Sussmuth, and G. Jung. 1997. J Exp Med 185:1951). Pam2Cys has the structure of Formula (II):

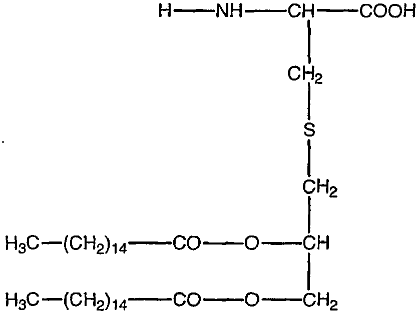

Pam2Cys is reported to be a more potent stimulator of splenocytes and macrophages than Pam3Cys (Metzger et al., J Pept. Sci 1, 184, 1995; Muhlradt et al., J Exp Med 185, 1951, 1997; and Muhlradt et al., Infect Immun 66, 4804, 1998).

Dendritic cells (DCs) are a rare population of antigen presenting cells (APCs) uniquely capable of stimulating primary immune responses, and a strong interest has developed in their use in cancer immunotherapies (Fong et al, Annu. Rev. Immunol. 18, 245, 2000). Attempts to harness the capacity of DCs to stimulate potent immune responses have hitherto focused primarily on procedures involving the manipulation of DCs *ex vivo.* This approach often requires that DCs be isolated from a patient, expanded in numbers, loaded with antigen (Ag) (Heiser, A. et al., J. Immunol. 166,2953,2001; Gatza et al., J. Immunol. 169, 5227, 2002; Timmerman et al., Blood 99, 1517, 2002; Marten et al., Mol. Immunol. 39, 395, 2002), and then be re-introduced into the patient. While this procedure is simple in principle, there are difficulties associated with isolation and culture of such a rare cell population (Inaba et a., J. Exp. Med. 172, 631, 1990; Wilson et al., P.N.A.S. USA 9,4784, 2000). Clearly, strategies that deliver Ags directly to DCs *in vivo,* and that can elicit an appropriate immune response, have enormous clinical potential.

DCs originate from progenitors in the bone marrow and migrate as immature cells to peripheral tissues where they internalise Ag and undergo a complex maturation process. Ag is internalised via a number of surface receptors, including the complement receptors (e.g., CD11c/CD18) and the endocytic receptors (e.g., DEC-205, DC-SIGN and Toll-like receptors). During Ag acquisition, immature DCs also may receive "danger signals", in the form of pathogen-related molecules such as bacterial cell wall lipopolysaccharide (LPS), or inflammatory stimuli via cytokines such as IFN-γ. DCs then migrate to the secondary lymphoid organs, maturing to become competent APCs (Guermonprez et al., Annu. Rev. Immunol. 20, 61, 2002). Receptors such as CD11c/CD18, DEC-205, DC-SIGN and Toll-like receptors play a crucial role in the process of Ag capture and presentation, and are expressed primarily on DCs.

In International Application No. PCT/AU00/00397 (Publication No. WO 00/64471) there is described a method of modifying biological or synthetic membranes or liposomes for the purposes of altering immunity, or for the targeting of drugs and other agents to a specific cell type or tissue when the modified biological or synthetic membranes or liposomes are administered *in vivo.* Modification of the membranes or liposomes is achieved by the incorporation or attachment of metal chelating groups, thereby allowing engraftment of one or more targeting molecules possessing a metal affinity tag.

In International Application No. PCT/AU2004/001125 (Publication No. WO 2005/01861) there is disclosed a composition for modulating immunity by the in *vivo* targeting of an antigen to dendritic cells, the composition comprising:
a preparation of antigen-containing membrane vesicles or antigen containing liposomes having on the surface thereof a plurality of metal chelating groups; and
a ligand for a receptor on said dendritic cells, said ligand being linked to a said metal chelating group via a metal affinity tag on said ligand; wherein,
said antigen-containing vesicles or liposomes include an immunomodulatory factor.

Chikh, G.G. et al. (Biochimica et Biophysica Acta 1567, 204-212, 2002) describes that recombinant proteins and synthetic peptides containing poly-histidine residues can be specifically bound to liposomes containing a metal ion chelating agent

### SUMMARY OF THE INVENTION

In a first aspect, the present invention provides an adjuvanting material, the adjuvanting material comprising a lipid dendritic cell targeting moiety to which is covalently linked a metal chelating group, as defined in the claims.

In a second aspect, the present invention provides an immunogenic composition comprising (a) a lipid dendritic cell targeting moiety to which is covalently linked a metal chelating group; (b) an antigen comprising a metal affinity tag; and optionally (c) metal ions, whereby the antigen is linked to the lipid dendritic cell targeting moiety via the interaction between the metal affinity tag and the metal chelating group, as defined in the claims.

### BRIEF DESCRIPTION OF THE FIGURES

**Figure 1** shows a construct comprising an adjuvant material according to a preferred embodiment of the present invention.
**Figure 2** shows a schematic illustrating a strategy for producing 3NTA-PEG-mal-Cys-Lys₈-Ser-Pam2Cys.

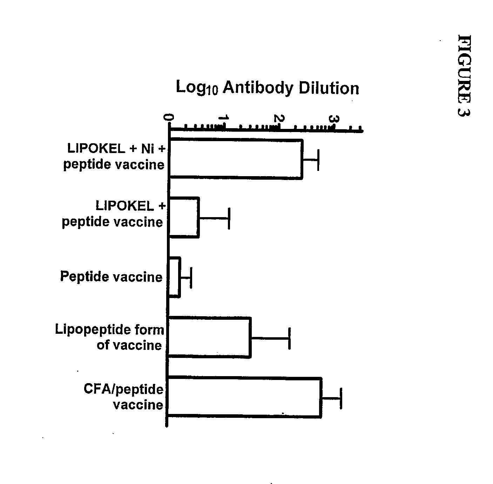

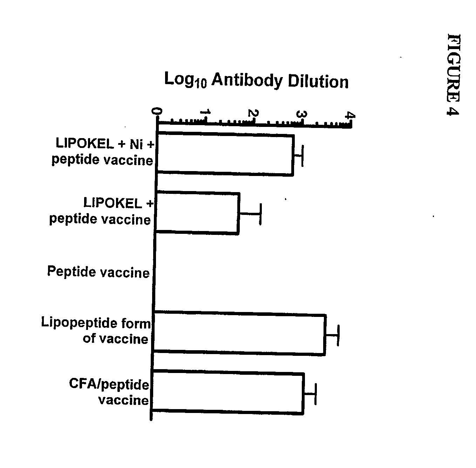

**Figures 3** **and** **4** show an antibody response to a His-tagged peptide vaccine delivered using an adjuvanting material according to the present invention. The material is referred to as LIPOKEL. LIPOKEL comprises the lipid moiety P₂CSK₈C coupled to 3NTA via the heterobifunctional linker molecule N-Succinimidyl 6-maleimidocaproate (MCS). Mice were given two doses of LIPOKEL co-admixed with HIS₆-ALNNRFQIKGVELKS-HWSYGLRPG in the presence or absence of nickel at week 0 and week 3. Control mice received HIS₆-ALNNRFQIKGVELKS-HWSYGLRPG alone, lipidated form of the peptide vaccine, or HIS₆-ALNNRFQIKGVELKS-HWSYGLRPG emulsified in Freund's Adjuvant respectively in the same schedule. The first dose was 20 nmoles per mouse and the second dose was 5 nmoles. Mice were bled at week 3 and week 4. ELISA was performed on sera from mice after one (Figure 3) or two (Figure 4) doses of vaccine.

### DETAILED DESCRIPTION OF THE INVENTION

In a first aspect, the present invention provides an adjuvanting material, the adjuvanting material comprising a lipid dendritic cell targeting moiety to which is covalently linked a metal chelating group, as claimed.

In a second aspect, the present invention provides an immunogenic composition comprising (a) a lipid dendritic cell targeting moiety to which is covalently linked a metal chelating group; (b) an antigen comprising a metal affinity tag; and optionally (c) metal ions, whereby the antigen is linked to the lipid dendritic cell targeting moiety via the interaction between the metal affinity tag and the metal chelating group, as claimed.

The lipid dendritic cell targeting moiety is Pam2Cys (S-(2,3-dipalmitate-propyl)cysteine) or Pam3Cys (N-palmitoyl-S-[2,3-bis(palmitoyloxy)propyl]cysteine).

It is particularly preferred that the lipid is Pam2Cys which has been shown to target TLR-2 receptors on dendritic cells (Jackson et al, PNAS, 101, 15440-15445, 2004).

Suitable metal chelating groups are known to those skilled in the art. Preferably, the metal chelating group is a carboxylic acid-based metal chelating group. For instance, the metal chelating group can be selected from 3-NTA (trinitrilotriacetic acid); N,N-bis(carboxymethyl)glycine (NTA) and its derivatives such as N-(5-amino-1-carboxypentyl)iminodiacetic acid; diethylene triamine pentaacetic acid (DTPA) and its derivatives; *N⁴*,*N^{α}*,*N^{α}*,*N^{ε}*,*N^{ε}*-[pentakis(carboxymethyl]-2,6-diamino-4-azahexanpoic hydrazide; ethylenedinitrilotetraacetic acid (EDTA) and its derivatives such as aminobenzyl-EDTA and isocyanabenzyl-EDTA; ethylenediaminedisuccinic acid (EDDS) and its derivatives; 1,4,7,10-tetraazacyclododecane-N,N',N",N"'-tetraacetic acid (DOTA) and its derivatives; and other carboxylic acid-based metal chelating moieties.

The metal chelating group is preferably 3-NTA.

In a preferred form of the second aspect, the immunogenic composition further comprises metal ions. The present inventors have found that the immunogenic compositions of the present invention provoke an immunogenic response in the absence of metal ions in the composition. Without being bound by theory, the present inventors consider that the immunogenic response is a result of the antigen being linked to the lipid dendritic cell targeting moiety by virtue of the presence of adventitious metal ions in the system to which the composition is administered. The present applicant has found that the immune response elicited by the composition is improved when metal ions are present in the immunogenic composition. Preferably, the metal ions are selected from the group consisting of Ni²⁺, Zn²⁺, Co²⁺ and Cu²⁺.

The antigen can be any suitable immunogenic protein, lipoprotein, or glycoprotein of a virus, prokaryotic or eukaryotic organism, including but not limited to an antigen derived from a mammalian subject or a bacterium, fungus, protozoan, or parasite that infects said subject. Idiotypic and anti-idiotypic B cell epitopes against which an immune response is desired are specifically included, as are lipid-modified B cell epitopes. Alternatively, the B cell epitope may be a carbohydrate antigen, such as, for example, an ABH blood group antigen, transplantation antigen (eg. Gal alpha1-3Gal beta1-4GlcNAc; Sandrin et al., Proc. Natl. Acad. Sci. USA 90, 11391-11395, 1993; Galili et al., Proc. Natl. Acad. Sci. USA 84, 1369-1373, 1987; Schofield et al., Nature 418: 785-789, 2002) or a conjugate thereof.

Preferred antigens from parasites are those associated with leishmania, malaria, trypanosomiasis, babesiosis, or schistosomiasis. Preferred virus antigens are derived from Hepatitis viruses, Rotaviruses, Herpes viruses, Corona viruses, Picornaviruses (eg. Apthovirus), Respiratory Syncytial virus, Influenza Virus, Parainfluenza virus, Adenovirus, Pox viruses, Bovine herpes virus Type I, Bovine viral diarrhea virus, Bovine rotaviruses, Canine Distemper Virus (CDV), Equine Rhinitis A Virus (ERAV); Equine Rhinitis B Virus (ERBV); Foot and Mouth Disease Virus (FMDV), Measles Virus (MV), Human Immunodeficiency Viruses (HIV), Feline Immunodeficiency Viruses (FIV), Epstein-Barr virus (EBV), and the like. Preferred bacterial antigens include those derived from Pasteurella, Actinobacillus, Haemophilus, Listeria monocytogenes, Mycobacterium, Staphylococcus, E. coli, Shigella, Salmonella and the like. Preferred antigens from mammalian subjects are derived from and/or are capable of generating an immune response against at least one tumor antigen.. Tumor antigens are usually native or foreign antigens, the expression of which is correlated with the development, growth, presence or recurrence of a tumor. In as much as tumor antigens are useful in differentiating abnormal from normal tissue, they are useful as a target for therapeutic intervention. Tumor antigens are well known in the art. Indeed, several examples are well-characterized and are currently the focus of great interest in the generation of tumor-specific therapies. Non-limiting examples of tumor antigens are carcinoembryonic antigen (CEA), prostate specific antigen (PSA), melanoma antigens (MAGE, BAGE, GAGE), and mucins, such as MUC-1.

Alternatively, the antigen from a mammalian subject is derived from zona pellucida protein such as ZP3 (Chamberlin and Dean Proc. Natl. Acad. Sci.(USA) 87, 6014-6018, 1990) or ZP3a (Yurewicz et al., Biochim. Biophys. Acta 1174,211-214,1993)] of humans or other mammals such as pigs. Particularly preferred antigens within this category include amino acid residues 323-341 of human ZP3 (Chamberlin and Dean Proc. Natl. Acad. Sci.(USA) 87, 6014-6018, 1990); amino acid residues 8-18 or residues 272-283 or residues 319-330 of porcine ZP3a (Yurewicz et al., Biochim. Biophys. Acta 1174, 211-214, 1993).

Further preferred antigens from a mammalian subject are derived from and/or capable of generating antibodies against a peptide hormone, such as, for example, a satiety hormone (eg. leptin), a digestive hormone (eg. gastrin), or a reproductive peptide hormone [eg. luteinising hormone-releasing hormone (LHRH), follicle stimulating hormone (FSH), luteinising hormone (LH), human chorionic gonadotropin (hCG; Carlsen et al., J. Biol. Chem. 248, 6810-6827, 1973), or alternatively, a hormone receptor such as, for example, the FSH receptor (Kraaij et al., J. Endocrinol. 158, 127-136, 1998). Particularly preferred epitopes within this category include the C-terminal portion (CTP) of b-hCG that is antigenically non cross-reactive with LH (Carlsen et al., J. Biol. Chem. 248, 6810-6827, 1973).

In a further preferred embodiment the antigen is a polytope which includes a number of different CTL epitopes.

Preferred antigens for particular viruses and organisms are listed below:

| **Virus** | **Antigen** |
|---|---|
| Human papilloma virus | E6E7 proteins |
| Influenza | M protein |
| Hepatitis B | hepatitis B small antigen (HBsAg) |
| Human immunodeficiency virus | gp120, gp41 |
| Herpes simplex | gB |

| **Organism** | **B subunit from toxins** |
|---|---|
| *Bacillus anthracis* | lethal factor |
| *Bordetella pertussis* | adenylate cyclase |
| *Bordetella pertussis* | pertussis toxin |
| *Clostridium tetani* | tetanus toxin |
| *Corynebacterium diphtheriae* | diphtheria toxin |
| Enterohaemorrhagic *E. coli* | Shiga toxin |
| Enterotoxigenic *E. coli* | heat-labile enterotoxin |
| *Vibrio cholerae* | cholera toxin |
| **Other Antigens** | ricin |

The metal affinity tag is preferably hexahistidine but can be a polyhistidine ranging from 4-16 histidine residues or a histidine-rich peptide that has affinity for a metal chelate, eg, histidine-proline-rich repeat peptides of mammalian histidine-rich glycoprotein (Hulett and Parish, Immunol. Cell Biol. 70, 280-287, 2000).

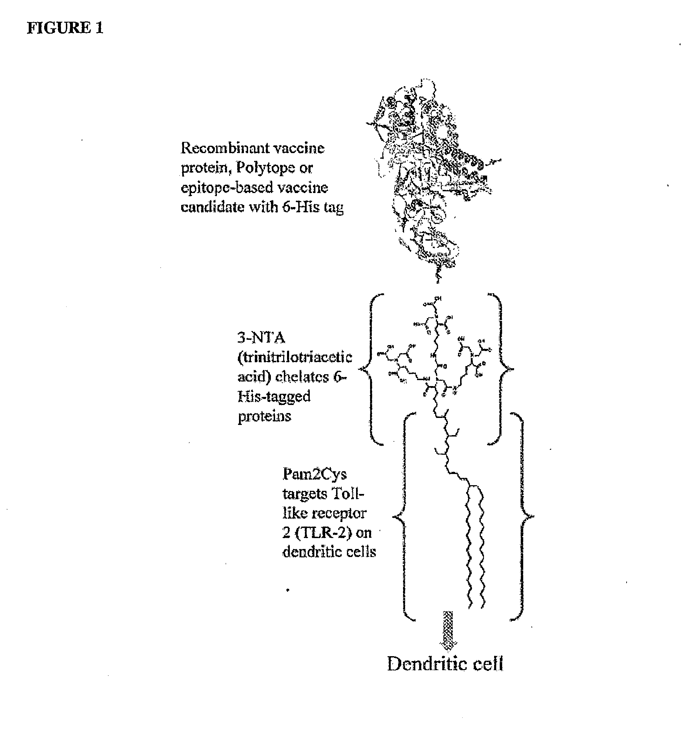

In a preferred embodiment, as shown in Figure 1, a construct according to the present invention includes Pam2Cys, a lipid which targets the Toll-like receptor 2 (TLR-2) on dendritic cells. 3-NTA is covalently attached to the Pam2Cys. The antigen is a 6-His tagged protein wherein the protein can be a recombinant vaccine protein, carbohydrate, polytope or epitope-based vaccine with a 6-His tag. The 3-NTA (trinitrilotriacetic acid) chelates to the 6-His tag so as to couple the Pam2Cys to the antigen whereby the construct of the preferred embodiment is formed.

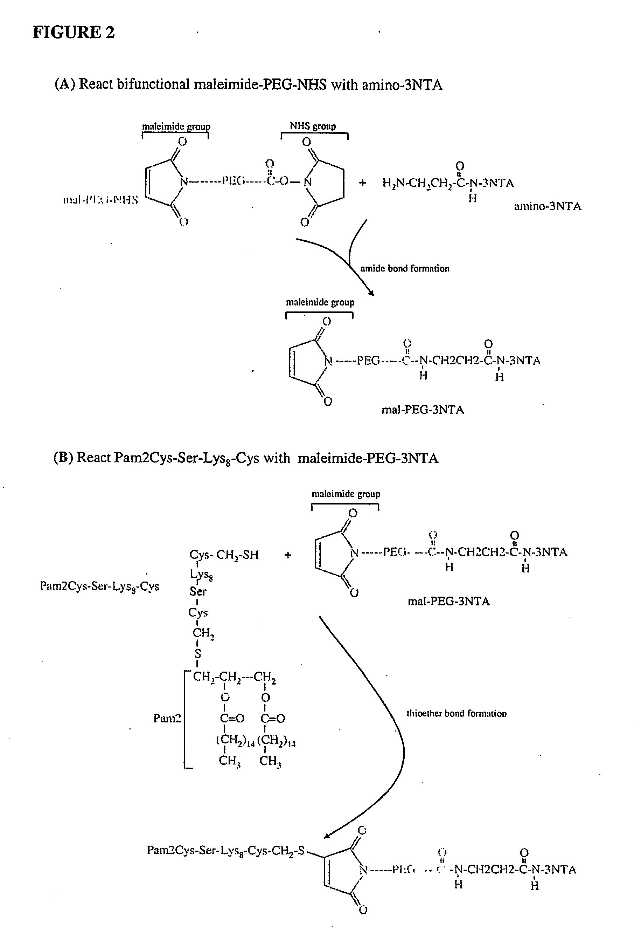

Figure 2 shows a schematic illustrating a strategy for generating 3NTA-PEG-Pam2Cys. As is described above this construct has great potential for serving as a generic vaccine by allowing increased scope for antigen delivery to DCs simply by varying the 6-His-tagged antigen associated with the construct through the 3NTA group. The construct shown here incorporates polyethylene glycol (PEG), which serves as a bridge linking 3NTA and Pam2Cys and, importantly, lends 'stealth-like' properties to the molecule (for improving in vivo efficacy of the product). PEG (Nektar Therapeutics), derivatised with a maleimide and an N-hydroxylsuccinimide-group (mal-PEG-NHS), provides a heterobifunctional cross-linker which allows coupling to thiol and amino groups, respectively. The 3NTA contains a functional amino group. The first reaction (A) shows the condensation reaction between the amino group of amino-3NTA and the NHS-group of mal-PEG-NHS to form an amide bond, producing mal-PEG-3NTA. (B) shows the thiol alkylation reaction between the maleimide group of mal-PEG-3NTA and the sulphydryl group of the terminal cysteine residue in Pam2Cys, resulting in the formation of a thioether bond. (A) and (B) may be carried out sequentially, in any order, or simultaneously. Alternatively the Pam2Cys and amino-3NTA can be coupled without the PEG spacer using a 'maleimido-succinimidyl' heterobifunctional cross-linker, such as sulfo-SMPB (Pierce), following the same principles of chemistry shown here. In a preferred form, the heterobifunctional cross-linker is N-succinimidyl 6-maleimidocaproate

As will be recognised by those skilled in this field the adjuvanting material is ideally suited for use with recombinant proteins or peptides which include a 6-His tag. The material of the present invention enables an antigen which includes a metal affinity tag to be readily coupled to a dendritic cell targeting lipid thereby increasing the immunogenicity of the antigen. This is particularly useful where the antigen is an expressed recombinant protein as these molecules are often produced with a 6-His tag for purification. These molecules can be simply reacted with the adjuvanting material of the present invention to yield the immunogenic composition of the second aspect of the present invention.

In order that the nature of the present invention may be more clearly understood, preferred forms thereof will now be described with reference to the following non-limiting examples.

### EXAMPLE 1

### MATERIALS AND METHODS

### 1. Chemicals

Unless otherwise stated chemicals were of analytical grade or its equivalent. N,N'-dimethylformamide (DMF), piperidine, trifluoroacetic acid (TFA), O'benzotriazole-N,N,N',N'-tetramethyluronium hexafluorophosphate (HBTU), 1-hydroxybenzotriazole (HOBt) and diisopropylethylamine (DIPEA) and diisopropylcarbodiimide (DIPCDI) were obtained from Auspep Pty. Ltd., Melbourne, Australia and Sigma-Aldrich Pty. Ltd., Castle Hill, Australia. Dichloromethane (DCM) and diethylether were from Merck Pty Ltd. (Kilsyth, Australia). Phenol and triisopropylsilane (TIPS) were from Aldrich (Milwaulke, WI) and trinitrobenzylsulphonic acid (TNBSA) and diaminopyridine (DMAP) from Fluka; 1,8-diazabicyclo[5.4.0]undec-7-ene (DBU) was obtained from Sigma and palmitic acid was from Fluka. The solid support TentaGel S RAM was from Rapp Polymere GmbH, Tubingen, GERMANY. O - (N-Fmoc-2-aminoethyl) -O'-(2-carboxyethyl) -undecaethylene glycol (Fmoc-PEG) was obtained from Novabiochem, Merck Biosciences, Switzerland. The heterobifunctional linker molecule N-Succinimidyl 6-maleimidocaproate (MCS) was from Fluka Biochemika, Switzerland. 3NTA was produced essentially as described in WO 2005/018610. NTA was purchased from Dojindo, Japan.

### 2. Synthesis of peptide vaccines

The peptide vaccine, His₆-**ALNNRFQIKGVELKS-HWSYGLRPG** comprises a 6 histidine residue, T helper cell epitope **ALNNRFQIKGVELKS** and a B cell epitope **HWSYGLRPG.** The T helper cell epitope is from the light chain (HA2) of influenza virus hemagglutinin and the B cell epitope is luteinising hormone releasing hormone (LHRH). The peptide vaccine was synthesized as a contiguous sequence by conventional solid-phase methodology using Fmoc chemistry. The general procedure used for the peptide synthesis has been described by Jackson et al., Vaccine 18, 355 (1999). The solid support TentaGel S RAM was used. The lipidated form of this peptide vaccine **ALNNRFQIKGVELKS-HWSYGLRPG** without six histidine residues was synthesised as described by Zeng, W. et al., Journal of Immunology 169,4905-4912.

### 3. Synthesis of LIPID Moieties

4 lipid moieties have been developed and synthesised:
(i) Pam₂CysSer (Lys)₈Cys
(ii) Pam₂CysSerSer (Lys)₈Cys
(ii) Pam₂CysSerSer PEG₁₀Cys
(iii) Pam₂CysSerSer FEG₂₀Cys

The lipid moieties were assembled by conventional solid-phase methodology using Fmoc chemistry. The general procedure used for the peptide synthesis has been described by Jackson et al., Vaccine 18,355 (1999). The solid support TentaGel S RAM was used. Four-fold excess of the Fmoc amino acid derivatives were used in the coupling steps except for the coupling of Fmoc-PEG where only two-fold excess was used. The difference of the first two lipid moieties is that an extra serine is inserted after the 8 lysine residues.

Pam2Cys was coupled to peptides according to the methods described by Jones et al., Xenobiotica 5, 155 (1975) and Metzger et al., Int J Pept Protein Res 38, 545 (1991), with the following modifications:

### I. Synthesis of S-(2,3-Dihydroxypropyl)cysteine:

Triethylamine (6 g, 8.2 ml, 58 mmoles) was added to L-cysteine hydrochloride (3 g, 19 mmole) and 3-bromo-propan-1,2-diol (4.2 g, 2.36 ml, 27 mmole) in water and the homogeneous solution kept at room temperature for 3 days. The solution was reduced in vacuo at 40°C to a white residue which was boiled with methanol (100ml), centrifuged and the residue dissolved in water (5ml). This aqueous solution was added to acetone (300ml) and the precipitate isolated by centrifugation. The precipitate was purified by several precipitations from water with acetone to give S-(2,3-dihydroxypropyl)cysteine as a white amorphous powder (2.4 g, 12.3 mmol, 64.7%).

### II. Synthesis of N-Fluorenylmethoxycarbonyl-S-(2,3-dihydroxypropyl)-cysteine (Fmoc-Dhc-OH):

S-(2,3-dihydroxypropyl)cysteine (2.45 g, 12.6 mmole) was dissolved in 9% sodium carbonate (20 ml). A solution of fluorenylmethoxycarbonyl-N-hydroxysuccinimide (3.45 g, 10.5 mmole) in acetonitrile (20 ml) was added and the mixture stirred for 2 h, then diluted with water (240 ml), and extracted with diethyl ether (25 ml x 3). The aqueous phase was acidified to pH 2 with concentrated hydrochloric acid and was then extracted with ethyl acetate (70 ml x 3). The extract was washed with water (50 ml x 2) and saturated sodium chloride solution (50 ml x 2), dried over sodium sulfate and evaporated to dryness. Recrystallisation from ether and ethyl acetate at -20°C yielded a colourless powder (2.8 g, 6.7 mmole, 63.8%).

### III. Coupling of Fmoc-Dhc-OH to resin-bound peptide:

Fmoc-Dhc-OH (100mg, 0.24 mmole) was activated in DCM and DMF (1:1, v/v, 3 ml) with HOBt (36 mg, 0.24 mmole) and DICI (37 ul, 0.24 mmol) at 0 °C for 5 min. The mixture was then added to a vessel containing the resin-bound peptide (0.04 mmole, 0.25g amino-peptide resin). After shaking for 2 h the solution was removed by filtration and the resin was washed with DCM and DMF (3 x 30 ml each). The reaction was monitored for completion using the TNBSA test. If necessary a double coupling was performed.

### IV. Palmitoylation of the two hydroxy groups of the Fmoc-Dhc-peptide resin:

Palmitic acid (204 mg, 0.8 mmole), DICI (154 ul, 1 mmole) and DMAP (9.76 mg, 0.08 mmole) were dissolved in 2 ml of DCM and 1 ml of DMF. The resin-bound Fmoc-Dhc-peptide resin (0.04 mmole, 0.25 g) was suspended in this solution and shaken for 16 h at room temperature. The solution was removed by filtration and the resin was then washed with DCM and DMF thoroughly to remove any residue of urea. The removal of the Fmoc group was accomplished with 2.5% DBU (2 x 5mins).

All resin-bound peptide constructs were cleaved from the solid phase support with reagent B (88% TFA, 5% phenol, 2% TIPS, 5% water) for 2 hr, and purified by reversed phase chromatography as described by Zeng et al., Vaccine 18, 1031 (2000).

Analytical reversed phase high pressure liquid chromatography (RP-HPLC) was carried out using a Vydac C4 column (4.6 x 300 mm) installed in a Waters HPLC system and developed at a flow rate of 1ml/min using 0.1% TFA in H2O and 0.1% TFA in CH3CN as the limit solvent. All products presented as a single major peak on analytical RP-HPLC and had the expected mass when analysed by Agilent 1100 LC-MSD trap mass spectrometer.

### 4. Synthesis of LIPOKELs

LIPOKEL comprises the lipid moiety P₂CSK₈C coupled to 3NTA via the heterobifunctional linker molecule N-Succinimidyl 6-maleimidocaproate (MCS). Modified versions of LIPOKEL have been synthesized using the lipid moieties P₂CS₂PEG₁₀ and P₂CS₂PEG₂₀ discussed above.

| | |
|---|---|
| LIPOKEL: | Pam₂CysSerLys₈Cys-3NTA |
| LIPOKELP-10: | Pam₂CysSerSerPEG₁₀-3NTA |
| LIPOKELP-20: | Pam₂CysSerSerPEG₂₀-MCS-3NTA |

Coupling of lipid moieties to 3NTA was performed as follows:
1) The coupling of 3NTA to MCS was achieved by mixing equimolar amounts of 3NTA and MCS in phosphate-buffered acetonitrile, and incubating at room temperature for 2-3 hours. The identity of 3NTA-MCS was confirmed by MS, and the compound was purified by HPLC.
2) The coupling of lipid moieties of 3NTA-MCS was performed with equimolar amounts of 3NTA-MCS and lipid moiety in a solution comprising phosphate-buffered acetonitrile to which solid guanidine powder was added such that all reaction components were soluble. It was found that the reaction efficiency was greatly increased at pH 7.5 compared to pH 6.0. The identity of reaction products was confirmed by MS, and LIPOKEL, LIPOKELP-10 and LIPOKELP-20 were purified by HPLC. The mass spectrum of LIPOKEL was determined using a mass spectrometer Agilent series 1100 LC-MSD. The experimental mass of 3073.95 corresponds closely to the calculated mass of 3074.9 Da.

### 5. Animal study

Five groups of BALB/c mice were given two doses (20 nmole for the first dose followed by 5 nmole for the second dose) of LIPOKEL co-admixed with HIS₆-ALNNRFQIKGVELKS-HWSYGLRPG in the presence or absence of nickel, HIS₆-ALNNRFQIKGVELKS-HWSYGLRPG alone, the lipidated form of ALNNRFQIKGVELKS-HWSYGLRPG, or HIS₆-ALNNRFQIKGVELKS-HWSYGLRPG emulsified in Freund's Adjuvant (first dose in complete and second dose in incomplete) respectively at week 0 and 3. Mice were bled at week 3 and 4 and sera were prepared and anti-LHRH antibody responses were determined by ELISA (Fig. 3 and 4).

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

## Claims

1. An adjuvanting material, the adjuvanting material comprising a lipid dendritic cell targeting moiety to which is covalently linked a metal chelating group; wherein the lipid dendritic cell targeting moiety is Pam2Cys (S-(2,3-dipalmitate-propyl)cysteine) or Pam3Cys (N-palmitoyl-S-[2,3-bis(palmitoyloxy)propyl]cysteine).

2. The adjuvanting material according to claim 1, wherein the lipid dendritic cell targeting moiety is Pam2Cys.

3. The adjuvanting material according to claim 1 or 2, wherein the metal chelating group is a carboxylic acid-based metal chelating group.

4. The adjuvanting material according to claim 3, wherein the metal chelating group is 3-NTA.

5. The adjuvanting material according to any one of claims 1 to 4, wherein the lipid dendritic cell targeting moiety and the metal chelating group are covalently linked by a heterobifunctional cross-linker.

6. The adjuvanting material according to claim 5, wherein the heterobifunctional cross- linker is N-succinimidyl 6-maleimidocaproate.

7. An immunogenic composition comprising (a) a lipid dendritic cell targeting moiety to which is covalently linked a metal chelating group; (b) an antigen comprising a metal affinity tag; and optionally (c) metal ions, whereby the antigen is linked to the lipid dendritic cell targeting moiety via the interaction between the metal affinity tag and the metal chelating group; wherein the lipid dendritic cell targeting moiety is Pam2Cys (S-(2,3-dipalmitate-propyl)cysteine) or Pam3Cys (N-palmitoyl-S-[2,3-bis(palmitoyloxy)propyl]cysteine).

8. The immunogenic composition according to claim 7, wherein the lipid dendritic cell targeting moiety is Pam2Cys.

9. The immunogenic composition according to claim 7 or 8, wherein the metal chelating group is a carboxylic acid-based metal chelating group.

10. The immunogenic composition according to claim 9, wherein the metal chelating group is 3-NTA.

11. The immunogenic composition according to any one of claims 7 to 10, wherein the lipid dendritic cell targeting moiety and the metal chelating group are covalently linked by a heterobifunctional cross-linker.

12. The adjuvanting material according to claim 11, wherein the heterobifunctional cross- linker is N-succinimidyl 6-maleimidocaproate

13. The immunogenic composition according to any one of claims 7 to 12, wherein the immunogenic composition further comprises metal ions.

14. The immunogenic composition according to claim 13, wherein the metal ions are selected from the group consisting of Ni²⁺, Zn²⁺, Co²⁺ and Cu²⁺.

15. The immunogenic composition according to any one of claims 7 to 14, wherein the antigen is derived from and/or is capable of generating an immune response against at least one tumor antigen.

16. The immunogenic composition according to any one of claim 7 to 15, wherein the antigen is a polytope which includes a number of different CTL epitopes.

17. The immunogenic composition according to any one of claims 7 to 16, wherein the metal affinity tag is a polyhistidine ranging from 4-16 histidine residues.

18. The immunogenic composition according to claim 17, wherein the metal affinity tag is hexahistidine.

## Patentansprüche

1. Adjuvansmaterial, wobei das Adjuvansmaterial einen dendritische Zelle zielführenden Lipidrest umfasst, an den eine Metall-chelatierende Gruppe kovalent verknüpft ist, wobei der dendritische Zelle zielführende Lipidrest Pam2Cys (S-(2,3-Dipalmitatpropyl)cystein) oder Pam3Cys (N-Palmitoyl-S-[2,3-bis(palmitoyloxy)propyl]cystein) ist.

2. Adjuvansmaterial nach Anspruch 1, wobei der dendritische Zelle zielführende Lipidrest Pam2Cys ist.

3. Adjuvansmaterial nach Anspruch 1 oder 2, wobei die Metall-chelatierende Gruppe eine Carbonsäure-basierende Metall-chelatierende Gruppe ist.

4. Adjuvansmaterial nach Anspruch 3, wobei die Metall-chelatierende Gruppe 3-NTA ist.

5. Adjuvansmaterial nach einem beliebigen der Ansprüche 1 bis 4, wobei der dendritische Zelle zielführende Lipidrest und die Metall-chelatierende Gruppe durch einen heterobifunktionalen Crosslinker kovalent verknüpft sind.

6. Adjuvansmaterial nach Anspruch 5, wobei der heterobifunktionale Crosslinker N-Succinimidyl-6-maleimidcaproat ist.

7. Immunogene Zusammensetzung, umfassend (a) einen dendritische Zelle zielführenden Lipidrest, an den kovalent eine Metall-chelatierende Gruppe verknüpft ist; (b) ein einen Metallaffinitäts-Tag umfassendes Antigen; und gegebenenfalls (c) Metallionen, wobei das Antigen mit dem dendritische Zelle zielführenden Lipidrest über die Wechselwirkung zwischen dem Metallaffinitäts-Tag und der Metall-chelatie-renden Gruppe verknüpft ist; wobei der dendritische Zelle zielführende Lipidrest Pam2Cys (S-(2,3-Dipalmitatpropyl)cystein) oder Pam3Cys (N-Palmitoyl-S-[2,3-bis(palmitoyloxy)propyl]cystein) ist.

8. Immunogene Zusammensetzung nach Anspruch 7, wobei der dendritische Zelle zielführende Lipidrest Pam2Cys ist.

9. Immunogene Zusammensetzung nach Anspruch 7 oder 8, wobei die Metall-chelatierende Gruppe eine Carbonsäure-basierende Metall-chelatierende Gruppe ist.

10. Immunogene Zusammensetzung nach Anspruch 9, wobei die Metall-chelatierende Gruppe 3-NTA ist.

11. Immunogene Zusammensetzung nach einem beliebigen der Ansprüche 7 bis 10, wobei der dendritische Zelle zielführende Lipidrest und die Metall-chelatierende Gruppe durch einen heterobifunktionalen Crosslinker kovalent verknüpft sind.

12. Adjuvansmaterial nach Anspruch 11, wobei der heterobifunktionale Crosslinker N-Succinimidyl-6-maleimidcaproat ist.

13. Immunogene Zusammensetzung nach einem beliebigen der Ansprüche 7 bis 12, wobei die immunogene Zusammensetzung des Weiteren Metallionen umfasst.

14. Immunogene Zusammensetzung nach Anspruch 13, wobei die Metallionen ausgewählt sind aus der Gruppe, bestehend aus Ni²⁺, Zn²⁺, Co²⁺ und Cu²⁺.

15. Immunogene Zusammensetzung nach einem beliebigen der Ansprüche 7 bis 14, wobei das Antigen aus mindestens einem Tumor-Antigen abgeleitet ist und/oder zum Erzeugen einer Immunantwort gegen mindestens ein Tumor-Antigen fähig ist.

16. Immunogene Zusammensetzung nach einem beliebigen der Ansprüche 7 bis 15, wobei das Antigen ein Polytop ist, das eine Zahl an unterschiedlichen CTL-Epitopen einschließt.

17. Immunogene Zusammensetzung nach einem beliebigen der Ansprüche 7 bis 16, wobei der Metallaffinitäts-Tag ein Polyhistidin im Bereich von 4-16 Histidinresten ist.

18. Immunogene Zusammensetzung nach Anspruch 17, wobei der Metallaffinitäts-Tag Hexahistidin ist.

## Revendications

1. Matière adjuvante, la matière adjuvante comprenant une fraction lipidique de ciblage des cellules dendritiques à laquelle est lié de manière covalente un groupe chélatant les métaux ; dans laquelle la fraction lipidique de ciblage des cellules dendritiques est Pam2Cys (S-(2,3-dipalmitate-propyl)cystéine) ou Pam3Cys (N-palmitoyl-S-[2,3-bis(palmitoyloxy)propyl)cystéine).

2. Matière adjuvante selon la revendication 1, dans laquelle la fraction lipidique de ciblage des cellules dendritiques est Pam2Cys.

3. Matière adjuvante selon la revendication 1 ou 2, dans laquelle le groupe chélatant les métaux est un groupe chélatant les métaux à base d'acide carboxylique.

4. Matière adjuvante selon la revendication 3, dans laquelle le groupe chélatant les métaux est le 3-NTA.

5. Matière adjuvante selon l'une quelconque des revendications 1 à 4, dans laquelle la fraction lipidique de ciblage des cellules dendritiques et le groupe chélatant les métaux sont liés de manière covalente par un agent de réticulation hétérobifonctionnel.

6. Matière adjuvante selon la revendication 5, dans laquelle l'agent de réticulation hétérobifonctionnel est le 6-maléimidocaproate de N-succinimidyle.

7. Composition immunogène comprenant (a) une fraction lipidique de ciblage des cellules dendritiques à laquelle est lié de manière covalente un groupe chélatant les métaux ; (b) un antigène comprenant une étiquette présentant une affinité pour les métaux ; et éventuellement (c) des ions métalliques, de sorte que l'antigène est lié à la fraction lipidique de ciblage des cellules dendritiques par l'intermédiaire de l'interaction entre l'étiquette présentant une affinité pour les métaux et le groupe chélatant les métaux ; dans laquelle la fraction lipidique de ciblage des cellules dendritiques est Pam2Cys (S-(2,3-dipalmitate-propyl)cystéine) ou Pam3Cys (N-palmitoyl-S-[2,3-bis(palmitoyloxy)propyl)cystéine).

8. Composition immunogène selon la revendication 7, dans laquelle la fraction lipidique de ciblage des cellules dendritiques est Pam2Cys.

9. Composition immunogène selon la revendication 7 ou 8, dans laquelle le groupe chélatant les métaux est un groupe chélatant les métaux à base d'acide carboxylique.

10. Composition immunogène selon la revendication 9, dans laquelle le groupe chélatant les métaux est le 3-NTA.

11. Composition immunogène selon l'une quelconque des revendications 7 à 10, dans laquelle la fraction lipidique de ciblage des cellules dendritiques et le groupe chélatant les métaux sont liés de manière covalente par un agent de réticulation hétérobifonctionnel.

12. Matière adjuvante selon la revendication 11, dans laquelle l'agent de réticulation hétérobifonctionnel est le 6-maléimidocaproate de N-succinimidyle.

13. Composition immunogène selon l'une quelconque des revendications 7 à 12, dans laquelle la composition immunogène comprend en outre des ions métalliques.

14. Composition immunogène selon la revendication 13, dans laquelle les ions métalliques sont choisis dans le groupe constitué par Ni²⁺, Zn²⁺, Co²⁺ et Cu²⁺.

15. Composition immunogène selon l'une quelconque des revendications 7 à 14, dans laquelle l'antigène est dérivé de et/ou est capable de générer une réponse immunitaire contre au moins un antigène tumoral.

16. Composition immunogène selon l'une quelconque des revendications 7 à 15, dans laquelle l'antigène est un polytope qui comprend un certain nombre d'épitopes de CTL différents.

17. Composition immunogène selon l'une quelconque des revendications 7 à 16, dans laquelle l'étiquette présentant une affinité pour les métaux est une poly-histidine allant de 4 à 16 résidus d'histidine.

18. Composition immunogène selon la revendication 17, dans laquelle l'étiquette présentant une affinité pour les métaux est une hexahistidine.
